# EUROPÄISCHE PATENTSCHRIFT

(11) **EP 2 217 360 B1**
(45) Veröffentlichungstag und Bekanntmachung des Hinweises auf die Patenterteilung: **04.05.2016**
(21) Anmeldenummer: 08842996.4
(22) Anmeldetag: 13.10.2008
(51) Int. Cl.: B01J 2/18, A61K 9/00

(54) **VORRICHTUNG UND VERFAHREN ZUR KONTINUIERLICHEN HERSTELLUNG VON SPHÄRISCHEN PULVERAGGLOMERATEN**
APPARATUS AND PROCESS FOR CONTINUOUSLY PRODUCING SPHERICAL POWDER AGGLOMERATES
DISPOSITIF ET PROCÉDÉ POUR LA PRODUCTION CONTINUE D'AGGLOMÉRATS DE POUDRE SPHÉRIQUES

(30) Priorität: 18.10.2007 DE 102007049931
(43) Veröffentlichungstag der Anmeldung: 18.08.2010
(73) Patentinhaber: Harro Höfliger Verpackungsmaschinen GmbH, 71573 Allmersbach im Tal (DE)
(72) Erfinder: HARTMANN, Thorsten, 10785 Berlin (DE); MÜLLER, Bernd, W., 24220 Flintbek (DE); STECKEL, Hartwig, 24232 Schönkirchen (DE)
(74) Vertreter: Wasmuth, Rolf
(86) Internationale Anmeldenummer: PCT/EP2008/008655
(87) Internationale Veröffentlichungsnummer: WO 2009/052967

(56) Entgegenhaltungen:
- EP-A- 0 441 740
- US-A- 4 688 610

## Beschreibung

### Hintergrund der Erfindung

Mikronisierte Substanzen, d.h. pulverförmige Güter mit einer Partikelgrößenverteilung im Bereich von 1 bis 10 µm, finden heutzutage eine Vielzahl von Anwendungen. In allen Fällen bestehen jedoch weitgehende Probleme bezüglich technischer Handhabbarkeit und exakter Dosierbarkeit aufgrund einer hohen Agglomerationsneigung der Einzelpartikel, da deren Autoadhäsionskräfte, hier vor allem Van-der-Waals-Kräfte, die auf sie wirkende Gravitationskraft in erheblichem Maße übertreffen.

Eine wichtige Stellung nimmt die Mikronisierung von Pulvern im Bereich der inhalativen Anwendung pharmazeutischer Wirkstoffe ein, denn um eine ausreichende pulmonale Verfügbarkeit zu gewährleisten, müssen entsprechende Partikel einen Durchmesser von in etwa 1 bis 5 µm aufweisen. Gerade auf diesem Gebiet spielt auch die präzise Abfassung häufig sehr geringer Volumina eine wichtige Rolle. Eine gängige Methode um die hierfür erforderliche suffiziente Fließfähigkeit einer Zubereitung zu erzielen ist die Herstellung von so genannten interaktiven Pulvermischungen, d.h. das bewusste Binden von mikronisierten Wirkstoffpartikeln an einen inerten Trägerstoff. Hierbei handelt es sich in den meisten Fällen um Laktosepartikel in einem Partikelgrößenbereich von 50 bis 200 µm, wodurch die somit wirkstoffhaltige Mischung im Vergleich zum reinen mikronisierten Wirkstoff ein deutlich besseres Fließvermögen erlangt. Mittels geeigneter Pulverinhalatoren wird durch gezielte Luftverwirbelungen innerhalb des Gerätes der Wirkstoff wiederum vom, Träger getrennt und kann somit inhaliert werden, wobei der Träger selbst im Gerät und/oder Mund-/Rachenraum des Anwenders verbleibt. Hierbei ist aber der Regel die Bindungsneigung zwischen Wirkstoffpartikel und Träger so hoch, dass ein Großteil des Wirkstoffes an den Träger gebunden bleibt und somit (je nach Bauart) ungenutzt im Pulverinhalator verbleibt bzw. vom Anwender verschluckt wird, statt wie gewünscht die Lunge zu erreichen.

Auf der Suche nach Alternativen zur interaktiven Pulvermischung zeigte sich, dass die gezielte Agglomeration von mikronisierten Wirkstoffen eine Alternative zur interaktiven Pulvermischung mit Trägerpartikeln darstellen kann. Hierbei werden sphärische Agglomerate aus reinem Wirkstoff bzw. unter Zusatz von ebenfalls mikronisierten Hilfsstoff hergestellt, stabil genug für die technische Handhabung, aber wiederum labil genug um eine pulmonale Applikation durch Redispergierung in einem entsprechend geeignetem Pulverinhalator zu ermöglichen.

### Stand der Technik

Das Patent SE19930003214 19931001 beschreibt die Herstellung solcher als "Softpellets" bezeichneten Agglomerate, wobei Ausgangsagglomerate mittels Pressen des mikronisierten Pulvers durch ein Sieb erzeugt werden. Das Pulver wird hierbei mit einem Schneckenförderer zugeführt und die erzeugten Ausgangsagglomerate chargenweise in einem rotierenden Topf ausgerundet und anschließend durch ein Sieb klassiert, um den Anteil an Agglomeraten eines zuvor festgelegten Größenbereiches zu erhalten.

Im Unterschied dazu setzen die erfindungsgemäße Vorrichtung und das erfindungsgemäße Verfahren auf eine komplett unterschiedliche Art der Ausrundung. Statt der chargenweisen Produktion in Kleinstmengen (bis 500 g) in einem rotierenden Kessel wird hierfür eine schwingende Oberfläche eingesetzt.

Vorteile zeigen sich hierbei:
- in der Möglichkeit auf chargenweises Herstellen zu verzichten und stattdessen kontinuierlich zu produzieren
- unter gegebenen Bedingungen Erhöhung der Ausbeute durch gezieltere Ausrundung einzelner Ausgangsagglomerate
- Zeitersparnis durch kürzere Ausrundungszeit; in den im folgenden angegebenen Beispielen in der Regel zwischen 30 und 90 Sekunden statt der in SE19930003214 19931001 angegebenen 120 bis 1200 Sekunden

Zudem liegen die in SE19930003214 19931001 beschriebenen Agglomerate bezüglich ihrer Härte bzw. Bruchfestigkeit in einem Bereich von 0,5 bis 100 mN. Im Unterschied dazu ermöglichen die erfindungsgemäße Vorrichtung und das erfindungsgemäße Verfahren eine Herstellung von sphärischen Agglomeraten mit einer Härte bzw. Bruchfestigkeit von weniger als 0,5 mN.

Die in SE19930003214 19931001 beschriebene Methode findet sich in genauer bzw. ähnlicher Form auch in folgenden Patenten, womit sich diese allesamt wesentlich von der von uns beschriebenen Erfindung unterscheiden: GB20000012261 20000519*;* GB19940004945 19940315*;* SE19970000133 19970120*;* SE19970000134 19970120*;* SE19970000135 19970120*;* SE19970000136 19970120

Das Patent CH19900000266 19900129 beschreibt eine Vorrichtung und ein Verfahren zur Dosierung von Kleinstmengen schlecht fließender Pulver.

Im Unterschied hierzu richten sich die erfindungsgemäße Vorrichtung und das erfindungsgemäße Verfahren nicht auf Abfüllung bzw. Dosierung sondern vielmehr die Herstellung von gezielt ausgerundeten Pulveragglomeraten. Zudem ist bei der vorliegenden Erfindung vor allem die deutlich sphärische Morphologie der resultierenden Pulveragglomerate das Ziel.

Darüber hinaus betrifft die CH19900000266 19900129 lediglich eine Formoterol-Laktose-Mischung und offenbart nicht den universellen Charakter des erfindungsgemäßen Verfahrens und der erfindungsgemäßen Vorrichtung.

Eine Reihe weiterer Patente befasst sich ebenfalls mit der Abfüllung bzw. Förderung von (zum Teil agglomerierten) Substanzen. Sie unterscheiden sich alle deutlich von der vorliegenden Erfindung, da sich im Wesentlichen die Förderung/Dosierung, einen vollkommen andersartigen Aufbau der Vorrichtung und (falls es zur Anwendung von Schwingungen/Vibrationen kommt) durch Frequenzbereiche.

Im Einzelnen sind dies:

*Die* GB19950015340 19950726 beschreibt des Dosieren von Pulvern, wobei das abzufüllende Volumen mittels Vibration eines Zufuhrtrichters in einem Frequenzbereich von 1 bis 1000 Hz erreicht werden soll. Im Gegensatz zu dem erfindungsgemäßen Verfahren und der erfindungsgemäßen Vorrichtung bestehen abgesehen von dem ähnlichen Frequenzbereich keinerlei Übereinstimmungen.

*Die* US20030434009 2003050 beschreibt Apparatur zur Dosierung von Pulvern, bei der der Abfüllvorgang durch ein Vibrieren des Fülltrichters in einem Frequenzbereich von 10 Hz bis 1000000 Hz ermöglicht bzw. unterstützt werden soll. Es wird explizit erwähnt, dass hierbei ein Pulverfluss ohne jegliche Aggregation, d.h. Agglomeration, erzeugt werden soll.

*Die* US19970100437P 19970721 betrifft den Transport von Pulvern, wobei pulverführende Teile vertikal in einem Frequenzbereich von 1000 bis 180000 Hz und horizontal, d.h. seitlich, in einem Frequenzbereich von 50 bis 50000 Hz schwingen.

*Auch die* US19960638515 1996042 beschränkt sich auf den Transport und eine anschließende Dosierung von Pulvern. Die Vorrichtung weist ein oder zwei Siebe auf. Diese Siebe schwingen mit einer Frequenz im Bereich von 1 bis 500 Hz. Es wird keinerlei Wert auf die Morphologie hierdurch erzeugter Agglomerate gelegt, ferner sogar davon ausgegangen, dass lediglich eine undefinierte Mischung aus kleinen Agglomeraten und Individualpartikeln erzeugt wird.

*Die* US19850713697 19850319 *beschreibt* eine Vorrichtung, die den Transport und anschließendes Dosieren von Pulvern ermöglichen soll, wobei der Pulverfluss mittels einer vibrierenden Transportfläche gewährleistet sein soll. Es wird keinerlei Augenmerk auf entstehende Pulveragglomerate bzw. deren Morphologie gelegt.

*Auch die* SE20040000282 20040209 *betrifft* das Abmessen und Abfüllen von Pulvern. Lediglich zur Unterstützung des Abfüllvorganges werden hierbei Schwingungen in Form von Ultraschall oder ähnlichen Vibrationen eingesetzt.

Aus der EP 0 441 740 A1 sind noch eine Vorrichtung und ein Verfahren bekannt, demnach zunächst eine unkontrollierte Agglomeration von Pulverteilchen erfolgt, woran sich dann eine Dosierung mit der gewünschten Menge von Agglomerationen anschließt.

Die der vorliegenden Erfindung zugrunde liegende Aufgabe bestand darin, ein neues Verfahren und eine neue Vorrichtung zur kontinuierlichen Herstellung von spärischen Pulveragglomeraten bereitzustellen, die die Nachteile des Standes der Technik überwinden.

Diese Aufgabe wird erfindungsgemäß durch ein Verfahren gemäß Anspruch 1 bzw. eine Vorrichtung gemäß Anspruch 9 gelöst.

Bevorzugte Ausführungsformen sind Gegenstand der Unteransprüche.

Bei der vorliegenden Erfindung handelt es sich um eine Vorrichtung und ein Verfahren zur kontinuierlichen Herstellung von sphärischen Pulveragglomeraten. Die zu erzielenden Eigenschaften der resultierenden sphärischen Agglomerate sollen annähernd perfekte Rundheit, somit möglichst gute Fließfähigkeit, damit einhergehend gute Dosierbarkeit, ausreichende Stabilität für die technische Handhabung und schließlich eine zufrieden stellende Redispergierfähigkeit bei pulmonaler Applikation umfassen.

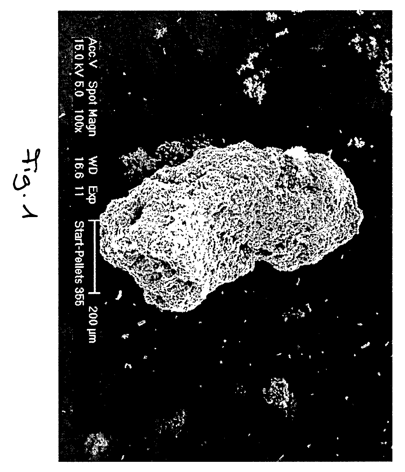

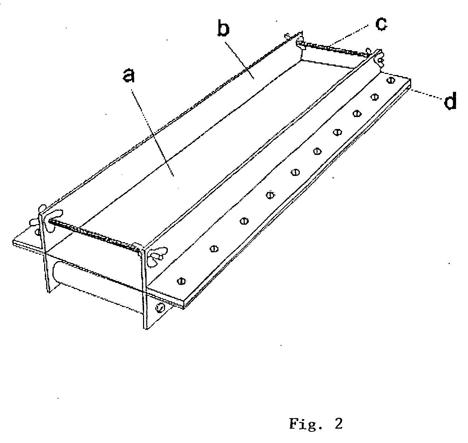

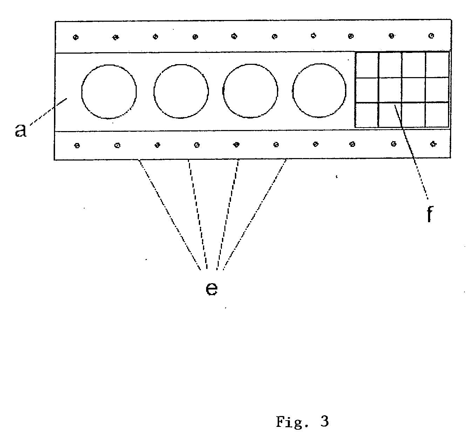

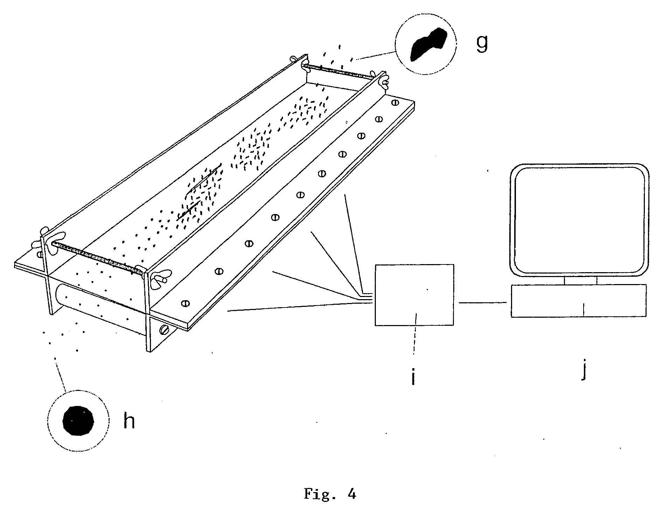

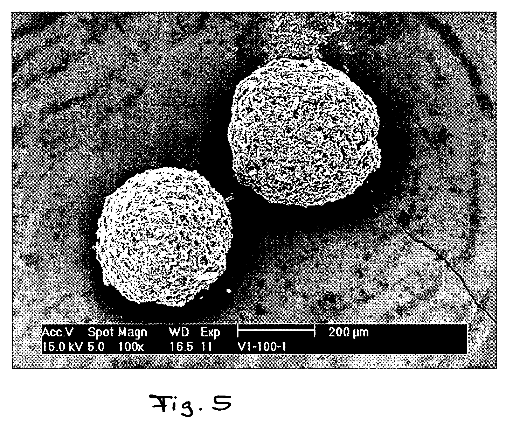

Hierzu wird das zu behandelnde Pulver, dessen Individualpartikel vorzugsweise mikronisiert, d.h. mit einem mittleren Durchmesser von 5 µm, vorliegen, mittels Pressen durch ein Sieb oder siebähnliche Vorrichtung zu morphologisch uneinheitlichen Ausgangsagglomeraten geformt [Fig. 1]. Hierbei kann es sich sowohl um reine Pulver, vorzugsweise pharmazeutische Wirkstoffe zur pulmonalen Applikation, als auch um Mischungen verschiedener Substanzen in. Pulverform, vorzugsweise Mischungen aus pharmazeutischen Wirkstoffen zur pulmonalen Applikation und einer bezüglich direkter pharmakologischen Wirkung inerten, vorzugsweise weitestgehend inerten, d.h. im Wesentlichen inerten, Füllsubstanz wie z.B. Laktose in verschiedensten Verhältnissen, handeln. Die Größe der Individualpartikel, aus denen die Ausgangsagglomerate gebildet werden, kann sich hierbei insgesamt über einen Bereich von 1 bis 50 µm erstrecken. Die Größe der Ausgangsagglomerate variiert in Abhängigkeit der Maschenweite des verwendeten Siebes oder siebähnlichen Vorrichtung zwischen 20 und 1000 µm. Die Wahl der Maschenweite und damit die Bestimmung der Größe sowohl der Ausgangs- als schließlich auch Endagglomerate sollte von den eingesetzten Substanzen abhängig gemacht werden. Dies heißt konkreter, dass Substanzen mit einer sehr hohen Autoadhäsionsneigung bei einer Endagglomeratsgröße von beispielsweise 50 µm trotz idealer Ausrundung nach wie vor ungenügende Fließfähigkeit aufweisen können und in diesem Fall eher größere Endagglomeratsdurchmesser angestrebt werden sollten. Andererseits können Mischungen mit höherem Anteil an größeren Partikeln bei der Wahl einer zu großen Maschenweite im Falle des Siebes oder der siebähnlichen Vorrichtung aufgrund ungenügender Adhäsionskräfte beispielsweise nur sehr labile Ausgangsagglomerate bilden, die sich folglich unter Umständen gar nicht ausrunden lassen sondern bereits bei dem Versuch wieder zerfallen.
Zur Ausrundung der Ausgangsagglomerate zu sphärischen Endagglomeraten dient ein in **[****Fig. 2** bis **4****]** veranschaulichter Aufbau. Kern der Vorrichtung stellt eine Metallfolie **[a]** dar, im vorliegenden Aufbau kaltgewalzter rostfreier Federbandstahl (1.4310 DIN X12 CrNi17 7) der Maße 15 cm x 50 cm x 0,1 mm, die an beiden Seiten mit Hilfe von jeweils 10 Schrauben zwischen zwei Aluminiumwinkel **[b]** gespannt ist. Zwei Metallstangen mit Flügelmuttern **[c]** bieten die Möglichkeit einer Regulation der Spannung der Metallfolie. Im Bereich der Kontaktstellen zu den Aluminiumwinkeln befindet sich ober- und unterhalb der Metallfolie eine Silikonschicht **[d],** im vorliegenden Aufbau 1 mm dick, die eine sichere Fixierung und zusätzliche Dämpfung gewährleistet.
Auf der Unterseite der Vorrichtung **[****Fig. 3****]** sind in regelmäßigen Abständen Frequenzgeber **[e]** direkt an die Metallfolie angebracht. Im vorliegenden Aufbau handelt es sich um vier Soundpad-NXT®-Folienlautsprecher, die mit kreisförmigen Stücken doppelseitigen Klebebandes an der Folie fixiert sind, wobei jedoch auch jede weitere Art von schwingungserzeugenden Bauteilen möglich ist. An einem der beiden Enden der Vorrichtung ist hierbei ein markierter Bereich **[f]** ausgespart.
Die fixierten Frequenzgeber werden mit Hilfe einer Kombination aus Regeleinheit **[i]** und Computer **[j]** in die gewünschte Schwingung versetzt **[****Fig. 4****],** die vorzugsweise eine Frequenz von 50 bis 150 Hz aufweist und aufgrund der festen Fixierung direkt auf die Metallfolie übertragen wird. Die gesamte Vorrichtung wird mit einer festgelegten Neigung - abhängig von der Länge der schwingenden Oberfläche, der angelegten Schwingungsfrequenz und resultierender Auslenkung sind hierbei theoretisch Winkel von 0 bis 89° denkbar, vorzugsweise jedoch Winkel zwischen 2 und 10° - auf einen festen Untergrund platziert. Gibt man nun die vorgeformten Ausgangsagglomerate **[g]** in den proximalen Bereich **[f]** auf, erfolgt aufgrund der schwingenden Oberfläche eine Ausrundung zeitgleich zur Bewegung zum distalen Ende der Vorrichtung. Hier können somit die sphärischen Endagglomerate **[h]** aufgefangen werden und stehen für weitere Verarbeitungsschritte wie einer eventuellen Klassierung oder direkter Abfüllung zur Verfügung. Auf diese Weise wird die kontinuierliche Produktion sphärischer Pulveragglomerate **[****Fig. 5****]** ermöglicht.

### Beispiele

1. Mikronisiertes Dinatriumcromoglycat (DSCG) wird mittels Pressen durch ein Sieb mit einer Maschenweite von 355 µm voragglomeriert. Rasterelektronenmikroskopische Aufnahmen dieser Ausgangsagglomerate sind in **[****Fig. 1****]** (siehe weiter oben) dargestellt. Diese Ausgangsagglomerate werden auf die bereits beschriebene Vorrichtung aufgebracht, in der Form, dass die Substanz direkt auf die Metallfolie aufgesiebt wird. Die Metallfolie besitzt wie beschrieben eine Gesamtlänge von 50 cm bei einer Breite von 15 cm. Hierbei schwingt die Folie rechnergesteuert mit einer Frequenz von 100 Hz. Die Neigung des Gesamtaufbaus zur Unterlage beträgt in etwa 7°. Am distalen Ende der Vorrichtung entnommene Agglomerate besitzen eine sphärische Morphologie, erkennbar in **[****Fig. 5****].** Anschließendes Klassieren mit Hilfe eines Siebes der Maschenweite 200 µm zeigt den lediglich marginalen Verlust in Form von Feinanteil, so dass sich eine Ausbeute im Bereich von 90 bis nahezu 100 % bezogen auf die Ausgangsmenge ergibt. Bei genaueren Bestimmungen der Agglomeratmorphologie mittels Bildanalyse (L2001, Lecö GmbH, Kirchheim, Deutschland) wird die sich anhand der rasterelektronenmikroskopischen Aufnahmen **[****Fig. 5****]** abzeichnende vollständige Ausrundung bestätigt Relevante Parameter stellen hierbei Aspect ratio (Längenverhältnis, im Idealfall = 1), Rundheit (RN = (4n*Fläche)/(Umfang)²; im Idealfall = 100, bzw. real = 95 aufgrund der Pixel-Matrix des Messsystems), Rauhigkeit (RG = Verhältnis Umfang / konvexer Umfang; im Idealfall = 1) und Breite (kürzester Feret-Durchmesser) dar. Im beschriebenen Beispiel lässt sich bei den resultierenden Agglomeraten ein Längenverhältnis von 1,16, bei einer Rundheit von 87,7 bzw. Rauhigkeit von 1,019 bestimmen. Die durchschnittliche Breite der Agglomerate liegt bei in etwa 366 µm. Aerodynamische Charakterisierungen der erhaltenen Agglomerate mittels Impaktorversuchen zeigen hierbei eine sehr zufrieden stellende Redispergierbarkeit in Einzelpartikel, die auf eine gute pulmonale Verfügbarkeit des behandelten Wirkstoffes DSCG schließen lässt.
2. Mikronisiertes Dinatriumcromoglycat (DSCG) wird wie in Beispiel 1 beschrieben behandelt, mit dem Unterschied, dass die resultierenden sphärischen Agglomerate zwei weitere Male auf die mit einer Frequenz von 100 Hz schwingende Metallfolie aufgegeben werden, d.h. somit die dreifache Wegstrecke auf der Folie zurücklegen. Erwartungsgemäß kommt es zu einer noch stärkeren Ausrundung, mit einem Längenverhältnis der resultierenden Agglomerate von 1,15, bei einer Rundheit von 88,6 bzw. Rauhigkeit von 1,016. Bei der aerodynamischen Charakterisierung zeigt sich ein ebenfalls ein Einfluss der geänderten Versuchsbedingungen in derart, dass der pulmonal verfügbare Anteil leicht abnimmt, jedoch nach wie vor im sehr zufrieden stellendem Rahmen liegt.
3. Mikronisiertes Dinatriumcromoglycat (DSCG) wird wie in Beispiel 1 beschrieben behandelt, mit dem Unterschied, dass die Metallfolie der Vorrichtung mit einer Frequenz von 50 Hz schwingt. Das Ergebnis liegt im vergleichbaren Rahmen, wobei die Qualität bezüglich Rundheit und Rauhigkeit etwas abnimmt, jedoch zu Gunsten einer verbesserten Redispergierbarkeit und damit pulmonalen Verfügbarkeit bei der aerodynamischen Charakterisierung.
4. Mikronisiertes Dinatriumcromoglycat (DSCG) wird wie in Beispiel 1 beschrieben behandelt, mit dem Unterschied, dass die Metallfolie nicht während des gesamten Herstellungsvorganges konstant mit einer Frequenz von 100 Hz schwingt, sondern die Frequenz moduliert wird. Genauer bedeutet dies, dass die Frequenz einen Bereich von 100 bis 20 Hz innerhalb eines Zeitraumes von einer Sekunde in Schritten von jeweils 1 Hz durchläuft. Nach Ablauf einer Sekunde springt die Frequenz sofort von 20 Hz zurück auf 100 Hz, um über den Zeitraum der nächsten Sekunde wiederum in Schritten von 1 Hz erneut 20 Hz zu erreichen. Diese Variante des Verfahrens liefert ebenfalls mit den anderen Beispielen vergleichbare Ergebnisse.
5. Mikronisiertes Budesonid wird ähnlich wie in Beispiel 1 beschrieben behandelt, mit vergleichbaren Ergebnissen als Resultat.
6. Mikronisiertes Fluticasonpropionat wird ähnlich wie in Beispiel 1 beschrieben behandelt, mit vergleichbaren Ergebnissen als Resultat.
7. Mikronisiertes Salbutamolsulfat wird ähnlich wie in Beispiel 1 beschrieben behandelt, mit vergleichbaren Ergebnissen als Resultat.
8. Eine Mischung aus mikronisiertem Formoterolfumarat und Laktose im Verhältnis 1:50 wird ähnlich wie in Beispiel 1 beschrieben behandelt, mit vergleichbaren Ergebnissen als Resultat.
9. Eine Mischung aus mikronisiertem Budesonid, mikronisiertem Formoterolfumarat und Laktose im Verhältnis 2:1:50 wird ähnlich wie in Beispiel 1 beschrieben behandelt, mit vergleichbaren Ergebnissen als Resultat.
10. Eine Mischung aus mikronisiertem Fluticasonpropionat, mikronisiertem Salmeterolxinafoat und Laktose im Verhältnis 2:1:50 wird ähnlich wie in Beispiel 1 beschrieben behandelt, mit vergleichbaren Ergebnissen als Resultat.
11. Pharmakologisch weitestgehend inerte Substanzen können ebenfalls wie in Beispiel 1 beschrieben behandelt werden. Hierbei liegt der Sinn logischerweise nicht darin, eine gute pulmonale Verfügbarkeit zu erreichen, sondern vielmehr darin, die Handhabbarkeit und Dosierbarkeit dieser Substanzen zu verbessern. Folglich liefert analog zu Beispiel 1 behandelte Laktose ebenfalls sphärische Agglomerate, die den genannten Kriterien entsprechen.
12. Analog zu Beispiel 10 erhält man vergleichbare Ergebnisse bei der Behandlung von Mannitol.

Die Figuren zeigen:
- **Fig. 1:**: Beispiel eines morphologisch uneinheitlichen Ausgangsagglomerates
- **Fig 2:**: Schematischer Aufbau der Vorrichtung, Oberseite
- **Fig. 3:**: Schematischer Aufbau der Vorrichtung, Unterseite
- **Fig. 4:**: Schematischer Aufbau der Vorrichtung, Gesamtansicht
- **Fig. 5:**: Beispiel zweier resultierender sphärischer Endagglomerate

## Patentansprüche

1. Verfahren zur kontinuierlichen Herstellung von sphärischen Pulveragglomeraten, **dadurch gekennzeichnet, dass** morphologisch unregelmäßige Ausgangsagglomerate aus mikronisierten, pulverförmigen Partikeln durch Aufgeben auf eine zur Schwingung angeregte geneigte Oberfläche kontinuierlich gezielt ausgerundet werden, wobei die morphologisch unregelmäßigen Ausgangsagglomerate mit einer Größe von 20 bis 1000 µm durch Pressen des zu behandelnden Pulvers mit einer Teilchengröße im Bereich von 1 bis 50 µm durch ein Sieb oder eine siebähnliche Vorrichtung mit einer Maschenweite von 20 bis 1000 µm erzeugt werden und die einzelnen nach der Behandlung resultierenden Endagglomerate eine sphärische Form aufweisen und im Größenbereich von 20 bis 1000 µm liegen.

2. Verfahren nach Anspruch 1, **dadurch gekennzeichnet, dass** es sich bei den zu behandelnden Pulvern um reine Monosubstanzen oder eine Mischung mehrerer Substanzen in Verhältnissen von 1 : 1 bis 1 : 1000 handelt.

3. Verfahren nach Anspruch 1 oder 2, **dadurch gekennzeichnet, dass** es sich bei den zu behandelnden Pulvern um pharmazeutische Wirkstoffe handelt, sowohl als Monösubstanz als auch in Form einer Mischung mit pharmakologisch inerten oder im Wesentlichen inerten Substanzen.

4. Verfahren nach Anspruch 1 oder 2, **dadurch gekennzeichnet, dass** es sich bei den zu behandelnden Pulvern um Mischung aus mehreren unterschiedlichen pharmazeutischen Wirkstoffen handelt.

5. Verfahren nach Anspruch 1 oder 2, **dadurch gekennzeichnet, dass** es sich bei den zu behandelnden Pulvern um Mischung aus einem oder mehreren unterschiedlichen pharmazeutischen Wirkstoffen und pharmakologisch inerten oder im Wesentlichen inerten Substanzen handelt.

6. Verfahren nach einem der vorhergehenden Ansprüche, **dadurch gekennzeichnet, dass** es sich bei den zu behandelnden Pulvern um pharmazeutische Wirkstoffe zur inhalativen Anwendung handelt.

7. Verfahren nach einem der vorhergehenden Ansprüche, **dadurch gekennzeichnet, dass** die morphologisch unregelmäßigen Ausgangsagglomerate durch Pressen des zu behandelnden Pulvers durch ein Sieb oder eine siebähnliche Vorrichtung mit einer Maschenweite von 100 bis 1000 µm, vorzugsweise 100 bis 500 µm erzeugt werden.

8. Verfahren nach einem der vorhergehenden Ansprüche, **dadurch gekennzeichnet, dass** die einzelnen nach der Behandlung resultierenden Endagglomerate eine sphärische Form aufweisen und im Größenbereich von 100 bis 1000 µm, vorzugsweise 100 bis 500 µm liegen.

9. Vorrichtung zur Durchführung des Verfahrens gemäß einem der Ansprüche 1 bis 8, **dadurch gekennzeichnet, dass** sie ein Sieb oder eine siebähnliche Vorrichtung mit einer Maschenweite von 20 bis 1000 µm aufweist, um das zu behandelnde Pulver mit einer Teilchengröße im Bereich von 1 bis 50 µm zur Erzeugung morphologisch unregelmäßiger Ausgangsagglomerate mit einer Größe von 20 bis 1000 µm hindurch zu pressen, und eine zur Schwingung angeregbare, geneigte Oberfläche aufweist, die insbesondere eine Folie aus Metall ist und vorzugsweise eine Dicke von 0,001 bis 10 mm, bevorzugter 0,025 bis 0,5 mm, sowie insbesondere eine Länge und eine Breite jeweils in einem Bereich von 10 bis 200 cm aufweist, um gezielt ausgerundete Endagglomerate herzustellen, die eine sphärische Form und im Größenbereich von 20 bis 1000 µm aufweisen.

10. Vorrichtung nach Anspruch 9, **dadurch gekennzeichnet, dass** die Erzeugung der. Schwingung mittels Frequenzgeber auf elektromechanischem Wege erfolgt.

11. Vorrichtung nach Anspruch 9, **dadurch gekennzeichnet, dass** die Erzeugung der Schwingung mittels Frequenzgeber auf mechanischem Wege erfolgt.

12. Vorrichtung nach Anspruch 9 oder 10, **dadurch gekennzeichnet, dass** die erzeugte Schwingung in einem Frequenzbereich von 10. bis 1000 Hz liegt, vorzugsweise zwischen 50 und 200 Hz.

13. Vorrichtung nach Anspruch 10, 11 oder 12, **dadurch gekennzeichnet, dass** alle angebrachten Frequenzgeber die gleiche Frequenz erzeugen oder mindestens ein Frequenzgeber eine zu den restlichen Frequenzgebern unterschiedliche Frequenz erzeugt.

14. Vorrichtung nach Anspruch 13, **dadurch gekennzeichnet, dass** es sich hierbei um eine während des gesamten Herstellungsdauer konstante Frequenz handelt.

15. Vorrichtung nach Anspruch 13, **dadurch gekennzeichnet, dass** die Frequenz während des Herstellungsvorganges im Rahmen des in Anspruch 13 angegebenen Frequenzbereiches, insbesondere in der Form, modulierbar ist, so dass statt einer festen Frequenz während des gesamten Herstellungsvorganges alternierend Frequenzbereiche durchlaufen werden.

16. Vorrichtung nach einem der Ansprüche 9 bis 15, **dadurch gekennzeichnet, dass** sie ein Sieb oder eine siebähnliche Vorrichtung mit einer Maschenweite von 100 bis 1000 µm, vorzugsweise 100 bis 500 µm aufweist.

## Claims

1. Method for continuously producing spherical powder agglomerates,
**characterised in that** morphologically irregular starting agglomerates of micronized powder-form particles are continuously rounded off by application to a surface induced to vibrate, wherein the morphologically irregular starting agglomerates with a size of from 20 to 1000 µm are produced by pressing the powder to be processed, having a particle size in the range of from 1 to 50 µm, through a sieve or a sieve-like apparatus having a mesh size of from 20 to 1000 µm, and the individual end agglomerates resulting after the processing have a spherical form and lie in the size range of from 20 to 1000 µm.

2. Method according to claim 1,
**characterised in that** the powders to be processed are pure mono-substances or a mixture of a plurality of substances in ratios of 1:1 to 1:1000.

3. Method according to claim 1 or 2,
**characterised in that** the powders to be processed are pharmaceutical agents, both as a mono-substance and also in the form of a mixture with pharmacologically inert or substantially inert substances.

4. Method according to claim 1 or 2,
**characterised in that** the powders to be processed are a mixture of a plurality of different pharmaceutical agents.

5. Method according to claim 1 or 2,
**characterised in that** the powders to be processed are a mixture of one or more different pharmaceutical agents and pharmacologically inert or substantially inert substances.

6. Method according to one of the preceding claims,
**characterised in that** the powders to be processed are pharmaceutical agents for inhalation.

7. Method according to one of the preceding claims,
**characterised in that** the morphologically irregular starting agglomerates are produced by pressing the powder to be processed through a sieve or a sieve-like device having a mesh size of 100 to 1000 µm, preferably 100 to 500 µm.

8. Method according to one of the preceding claims,
**characterised in that** the individual end agglomerates resulting after the processing have a spherical form and lie in the size range of from 100 to 1000 µm, preferably 100 to 500 µm.

9. Apparatus for carrying out the method according to one of claims 1 to 8,
**characterised in that** it has a sieve or a sieve-like device having a mesh size of 20 to 1000 µm in order to press the powder to be processed, having a particle size in the range of from 1 to 50 µm, to produce morphologically irregular starting agglomerates having a size of from 20 to 1000 µm, and has an inclined surface induced to vibrate, which is in particular a foil made of metal, and preferably has a thickness of from 0.001 to 10 mm, more preferably 0.025 to 0.5 mm, and in particular a length and a width respectively in the range of from 10 to 200 cm, in order to produce rounded-off end agglomerates of a spherical form and having a size range of from 20 to 1000 µm.

10. Apparatus according to claim 9,
**characterised in that** the vibration is generated by means of a frequency generator in an electrical- mechanical manner.

11. Apparatus according to claim 9,
**characterised in that** the vibration is generated by means of a frequency generator in a mechanical manner.

12. Apparatus according to claim 9 or 10,
**characterised in that** the generated vibration lies in a frequency range of from 10 to 1000 Hz, preferably between 50 and 200 Hz.

13. Apparatus according to claim 10, 11 or 12,
**characterised in that** all applied frequency generators generate the same frequency or at least one frequency generator generates a frequency that is different from the other frequency generators.

14. Apparatus according to claim 13,
**characterised in that** the frequency is constant throughout the whole production duration.

15. Apparatus according to claim 13,
**characterised in that** the frequency can be modulated during the production process within the scope of the frequency range indicated in claim 13, in particular in such a manner that, instead of a fixed frequency during the whole production process, frequency ranges are covered in an alternating manner.

16. Apparatus according to one of claims 9 to 15,
**characterised in that** it has a sieve or a sieve-like device having a mesh size of 100 to 1000 µm, preferably 100 to 500 µm.

## Revendications

1. Procédé pour la production continue d'agglomérats de poudre sphériques,
**caractérisé en ce que** des agglomérats de départ morphologiquement irréguliers et composés de particules de poudre micronisées sont arrondis en continu de manière appropriée en étant amenés sur une surface inclinée que l'on fait vibrer, étant précisé que les agglomérats de départ morphologiquement irréguliers d'une taille de 20 à 1000 µm sont produits par compression de la poudre à traiter, d'une taille de particules située dans la plage de 1 à 50 µm, à travers un tamis ou un dispositif du type tamis d'une largeur de mailles de 20 à 1000 µm, et que les agglomérats finis individuels obtenus après le traitement présentent une forme sphérique et sont situés dans la plage de taille de 20 à 1000 µm.

2. Procédé selon la revendication 1,
**caractérisé en ce qu'**il s'agit, pour les poudres à traiter, de substances monoconstituant pures ou d'un mélange de plusieurs substances dans des rapports de 1 : 1 à 1 : 1000.

3. Procédé selon la revendication 1 ou 2,
**caractérisé en ce qu'**il s'agit, pour les poudres à traiter, de matières actives pharmaceutiques, aussi bien sous la forme de substances monoconstituant que d'un mélange avec des substances pharmacologiquement inertes ou globalement inertes.

4. Procédé selon la revendication 1 ou 2,
**caractérisé en ce qu'**il s'agit, pour les poudres à traiter, d'un mélange de plusieurs matières actives pharmaceutiques différentes.

5. Procédé selon la revendication 1 ou 2,
**caractérisé en ce qu'**il s'agit, pour les poudres à traiter, d'un mélange d'une ou plusieurs matières actives pharmaceutiques différentes et de substances pharmacologiquement inertes ou globalement inertes.

6. Procédé selon l'une des revendications précédentes,
**caractérisé en ce qu'**il s'agit, pour les poudres à traiter, de matières actives pharmaceutiques à administrer par inhalation.

7. Procédé selon l'une des revendications précédentes,
**caractérisé en ce que** les agglomérats de départ morphologiquement irréguliers sont produits par compression de la poudre à traiter à travers un tamis ou un dispositif du type tamis d'une largeur de mailles de 100 à 1000 µm, de préférence de 100 à 500 µm.

8. Procédé selon l'une des revendications précédentes,
**caractérisé en ce que** les agglomérats finis individuels obtenus après le traitement présentent une forme sphérique et sont situés dans la plage de taille de 100 à 1000 µm, de préférence de 100 à 500 µm.

9. Dispositif pour la mise en oeuvre du procédé selon l'une des revendications 1 à 8,
**caractérisé en ce qu'**il comporte un tamis ou un dispositif du type tamis avec une largeur de mailles de 20 à 100 µm pour comprimer au travers la poudre à traiter, d'une taille de particules située dans la plage de 1 à 50 µm en vue de produire des agglomérats morphologiquement irréguliers d'une taille de 20 à 1000 µm, et une surface inclinée apte à vibrer qui est constituée en particulier par une feuille de métal et qui présente de préférence une épaisseur de 0,001 à 10 mm, plus spécialement de 0,025 à 0,5 mm, et en particulier une longueur et une largeur situées chacune dans une plage de 10 à 200 cm afin de produire des agglomérats finis, arrondis d'une manière appropriée, qui présentent une forme sphérique et sont situés dans la plage de taille de 20 à 1000 µm.

10. Dispositif selon la revendication 9,
**caractérisé en ce que** la production des vibrations se fait à l'aide d'un générateur de fréquence par voie électromécanique.

11. Dispositif selon la revendication 9,
**caractérisé en ce que** la production des vibrations se fait à l'aide d'un générateur de fréquence par voie mécanique.

12. Dispositif selon la revendication 9 ou 10,
**caractérisé en ce que** les vibrations produites sont situées dans une plage de fréquence de 10 à 1000 Hz, de préférence entre 50 et 200 Hz.

13. Dispositif selon la revendication 10, 11 ou 12,
**caractérisé en ce que** tous les générateurs de fréquence installés produisent la même fréquence, ou au moins un générateur de fréquence produit une fréquence différente par rapport aux autres générateurs de fréquence.

14. Dispositif selon la revendication 13,
**caractérisé en ce qu'**il s'agit d'une fréquence constante pendant toute la durée de production.

15. Dispositif selon la revendication 13,
**caractérisé en ce que** la fréquence est modulable pendant l'opération de production dans le cadre de la plage de fréquence indiquée dans la revendication 13, en particulier de telle sorte qu'au lieu d'une fréquence fixe pendant toute l'opération de production, des plages de fréquence sont appliquées en alternance.

16. Dispositif selon l'une des revendications 9 à 15,
**caractérisé en ce qu'**il comporte un tamis ou un dispositif du type tamis d'une largeur de mailles de 100 à 1000 µm, de préférence de 100 à 500 µm.
